# EUROPEAN PATENT SPECIFICATION

(11) **EP 4 212 105 B1**
(45) Date of publication and mention of the grant of the patent: **30.09.2026**
(21) Application number: 22201483.9
(22) Date of filing: 14.10.2022
(51) Int. Cl.: A61B 17/00, A61B 17/29, A61B 34/00, A61B 1/005, A61B 5/06, A61M 25/01

(54) **HYSTERESIS COMPENSATION CONTROL APPARATUS OF FLEXIBLE TUBE**
HYSTERESEKOMPENSATIONSSTEUERVORRICHTUNG FÜR FLEXIBLE RÖHRE
APPAREIL DE COMMANDE DE COMPENSATION D'HYSTÉRÉSIS DE TUBE FLEXIBLE

(30) Priority: 18.10.2021 KR 20210138056; 07.10.2022 KR 20220128324
(43) Date of publication of application: 19.07.2023
(73) Proprietor: ROEN Surgical, Inc., Daejeon 34051 (KR)
(72) Inventor: Lee, Dong-Ho, Daejeon (KR); Kim, Joon-Hwan, Daejeon (KR); Won, Jong-seok, Daejeon (KR); Kwon, Dong-Soo, Daejeon (KR)
(74) Representative: LLR

(56) References cited:
- WO-A1-2021/145490
- WO-A1-2021/173315
- US-A1- 2004 138 530
- NOROUZI-GHAZBI SOMAYEH ET AL: "A Switching Image-Based Visual Servoing Method for Cooperative Continuum Robots", JOURNAL OF INTELLIGENT SPRINGER NETHERLANDS, DORDRECHT, vol. 103, no. 3, 14 October 2021 (2021-10-14), XP037589436, ISSN: 0921-0296, [retrieved on 20211014], DOI: 10.1007/S10846-021-01435-W
- BAEK DONGHOON ET AL: "Hysteresis Compensator With Learning-Based Hybrid Joint Angle Estimation for Flexible Surgery Robots", IEEE ROBOTICS AND AUTOMATION LETTERS, vol. 5, no. 4, 1 October 2020 (2020-10-01), pages 6837 - 6844, XP093405141, ISSN: 2377-3774, Retrieved from the Internet <URL:https://ieeexplore.ieee.org/document/8989818> DOI: 10.1109/lra.2020.2972821

## Description

### BACKGROUND OF THE INVENTION

### Field of the Invention

The present invention relates to a hysteresis compensation control apparatus of a flexible tube and more specifically, to a hysteresis compensation control apparatus of a flexible tube for compensation control of hysteresis of a surgical instrument disposed in a channel of an overtube.

### Background Art

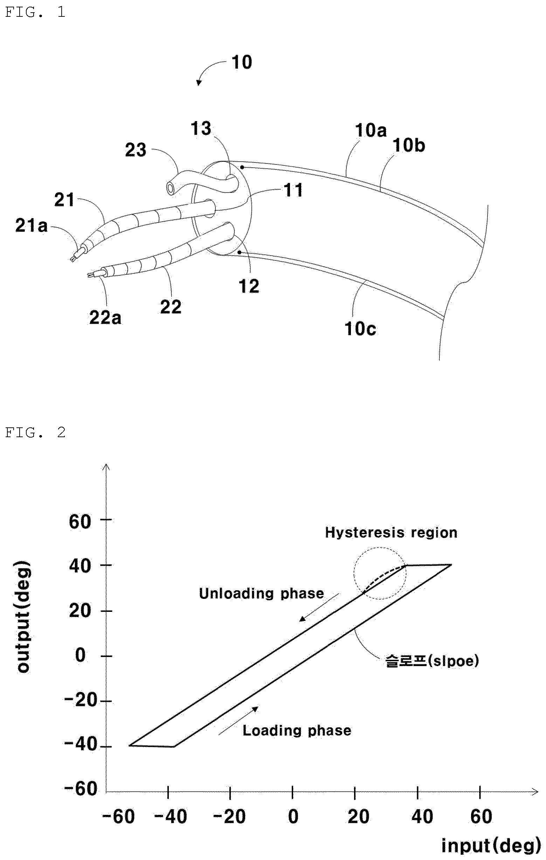

As illustrated in FIG. 1, a flexible overtube 10 includes a plurality of flexible surgical instruments 21 and 22. The plurality of flexible surgical instruments 21 and 22 are inserted into channels 11 and 12 provided in a body of the overtube 10. As an example, the first surgical instrument 21 is towed by first and second traction wires (not shown) to control position and orientation.

The flexible surgical instrument has a hysteresis section as illustrated in FIG. 2. That is, even if the same traction power is given, the hysteresis section is generated according to the shape of the flexible overtube or the surgical instrument. There is a disadvantage in that the overtube or the surgical instrument cannot be precisely controlled in the hysteresis generation section.

### PATENT LITERATURE

### Patent Documents

US 2019/0290109 WO 2021/173315 and WO2021/145490 disclose robotic surgical systems and/or instruments, and methods for operating the same.

### Non-patent Documents

NOROUZI-GHAZBI, Somayeh, et al. "A Switching Image-Based Visual Servoing Method for Cooperative Continuum Robots." Journal of Intelligent & Robotic Systems, Springer Netherlands, Dordrecht, 14 October 2021, vol. 103, no. 3, XP037589436, ISSN: 0921-0296, DOI: 10.1007/s10846-021-01435-w; and BAEK, Donghoon, et al. "Hysteresis Compensator With Learning-Based Hybrid Joint Angle Estimation for Flexible Surgery Robots." IEEE Robotics and Automation Letters, 1 October 2020, vol. 5, no. 4, pages 6837-6844, XP093405141, ISSN: 2377-3774, DOI: 10.1109/LRA.2020.2972821 disclose further prior art.

### SUMMARY OF THE INVENTION

Accordingly, the present invention has been made to solve the above-mentioned problems occurring in the prior arts, and it is an object of the present invention to provide a hysteresis compensation control apparatus of a flexible tube capable of precisely controlling a flexible surgical instrument even in a hysteresis section.

To accomplish the above object, according to the present invention, there is provided a hysteresis compensation control apparatus for a flexible overtube according to claim 1. Preferred embodiments are given in the dependent claims. Methods disclosed hereafter do not form part of the claimed invention.

Moreover, the image information for determining a current state of the surgical instrument is image information obtained by capturing a tip region of the surgical instrument inserted into a channel of a tube body and towed by a traction wire.

Furthermore, the input unit includes: a surgical instrument image capturing unit inserted into the channel of the tube body to capture an image of the surgical instrument; and a tension measuring unit for measuring traction force of a traction wire of the surgical instrument, wherein the image information of the tip region of the surgical instrument captured by the surgical instrument image capturing unit is transmitted to the mode switching determining unit to determine the current state of the surgical instrument.

Additionally, in the mode switching determining unit determines whether it is possible to control the image information-based flexible tube control mode on the basis of the image information of the tip region of the surgical instrument.

In addition, the hysteresis compensation control apparatus further includes: an image information learning unit for learning according to a predetermined learning model based on image information of the surgical instrument, and calculating an image error value by learning; and a tension information learning unit for learning according to a predetermined learning model based on the tension information of the surgical instrument, and calculating a tension error value by learning.

In another aspect of the present invention, there is provided a hysteresis compensation control apparatus including: a mode switching determining unit for generating a first control mode switching signal by determining a current state of a flexible surgical instrument on the basis of image information of the flexible surgical instrument or generating a second control mode switching signal by determining whether the control mode corresponds to a predefined tension control modeling on the basis of tension information of the flexible surgical instrument; an image mode switching unit for switching a control mode from a flexible tube control based on the tension information to a flexible tube control based on the image information; and a compensation control unit for compensatingly controlling the flexible surgical instrument having hysteresis characteristics by using a compensation error value generated on the basis of the tension information or the image information.

Moreover, the first control mode switching signal is generated when the tip region of the surgical instrument included in the image information determining the current state of the surgical instrument is not visible or is hidden by another surgical instrument.

Furthermore, the tension mode switching unit switches the control mode according to the first control mode switching signal.

Additionally, the second control mode switching signal is generated when there is no change in the load of the surgical instrument or when any one among the plurality of tension measurement values of the surgical instrument is zero and does not correspond to the tension control modeling.

In addition, the image mode switching unit switches the control mode according to the second control mode switching signal.

In a further aspect of the present invention, there is provided a hysteresis compensation control method including the steps of: compensatingly controlling a flexible surgical instrument having hysteresis characteristics on the basis of image information of a flexible surgical instrument; determining whether the surgical instrument is visible or not on the basis of image information for determining a current state of the surgical instrument; switching a control mode from an image information-based compensation control to a tension information-based compensation control when it is determined that the surgical instrument is not visible; and compensatingly controlling the flexible surgical instrument having the hysteresis characteristics on the basis of the tension information of the flexible surgical instrument.

Additionally, after the control mode is switched from the image information-based compensation control to the tension information-based compensation control, the hysteresis compensation control method further includes the steps of: determining whether the control mode corresponds to the predefined tension control modeling based on the tension information of the flexible surgical instrument; switching the control mode from the tension information-based compensation control to the image information-based compensation control when it is determined that the control mode does not correspond to the predefined tension control modeling; and compensatingly controlling the flexible surgical instrument having hysteresis characteristics on the basis of the image information of the flexible surgical instrument.

According to the present invention as described above, the hysteresis compensation control apparatus of a flexible tube and the method thereof can precisely control the flexible surgical instrument even in a hysteresis section.

### BRIEF DESCRIPTION OF THE DRAWINGS

The above and other objects, features and advantages of the present invention will be apparent from the following detailed description of the preferred embodiments of the invention in conjunction with the accompanying drawings, in which:
FIG. 1 is a diagram illustrating a flexible overtube according to an embodiment of the present invention;
FIG. 2 is a graph showing hysteresis characteristics or curves of a flexible surgical instrument according to an embodiment of the present invention;
FIG. 3 is a schematic diagram of a hysteresis compensation control apparatus of a flexible overtube according to a first embodiment of the present invention;
FIG. 4 is a view illustrating a process of switching a control mode from an image information-based hysteresis compensation control to a tension information-based hysteresis compensation control in sequential order according to the first embodiment of the present invention;
FIG. 5 is a graph illustrating a process of switching the control mode from the tension information-based hysteresis compensation control to the image information-based hysteresis compensation control in sequential order according to the first embodiment of the present invention after switching the control mode from the image information-based hysteresis compensation control to the tension information-based hysteresis compensation control;
FIG. 6 is a schematic diagram of a hysteresis compensation control apparatus of a flexible overtube according to a second embodiment of the present invention;
FIG. 7 is a graph illustrating a process of switching the control mode from an image information-based hysteresis compensation control to a tension information-based hysteresis compensation control in sequential order according to the second embodiment of the present invention after switching the control mode from a tension information-based hysteresis compensation control to an image information-based hysteresis compensation control;
FIG. 8 is a schematic diagram of a configuration for learning a hysteresis compensation model according to a third embodiment of the present invention; and
FIG. 9 is a schematic diagram of a configuration for a hysteresis compensation control according to the third embodiment of the present invention.

### DETAILED DESCRIPTION OF THE PREFERRED EMBODIMENT

A hysteresis compensation control apparatus of a flexible tube according to an embodiment of the present invention is a device for compensatingly controlling hysteresis generated in a flexible tube. In this instance, the flexible tube is a tube driven or towed by a wire, and is, for instance, a flexible overtube or a flexible surgical instrument or a flexible robot surgical instrument inserted into a channel of the flexible overtube. Hereinafter, a flexible tube 10 or flexible surgical instruments 21 and 22 will be described as an example of the flexible tube.

For the flexible overtube 10, the flexible surgical instrument is inserted into a flexible tube body and the channel of the flexible tube. The flexible overtube 10 is an apparatus which is inserted into a human body, and controls the position and orientation of the flexible tube and the surgical instrument through a traction wire to operate the human body.

As illustrated in FIG. 1, the flexible overtube or tube 10 includes an overtube body 10a having a flexible body, first, second, and third channels 11, 12 and 13 formed inside the overtube body 10a to have a predetermined diameter, and a surgical instrument and a camera unit inserted into each channel. As illustrated in FIG. 1, a first surgical instrument 21 is inserted into the first channel 11, a second surgical instrument 22 is inserted into the second channel 12, and the camera unit 23 is inserted into the third channel 13. However, the number of channels and the number of surgical instruments can be changed as needed. The camera unit 23 and the surgical instrument inserted into each channel protrude from the front end of the overtube body 10a. Accordingly, the camera unit 23 can capture the front side, and the surgical instruments can control the surgical operation for picking up or cut a tissue according to manipulation of the traction wire or a driving wire. A manipulation unit or a control unit (not shown) capable of manipulating the traction wire (not shown) is arranged at the rear end portion of the overtube body 10a. The traction wire is connected from the manipulation unit to the front end of the overtube body or the surgical instrument. Therefore, the position and orientation of the traction wire can be controlled by a user's manipulation. In this instance, as an example, in order to control the position and orientation of the flexible overtube body 10a, a first traction wire 10b and a second traction wire 10c are adjusted by a manipulation unit so that the position and orientation are changed according to the traction force. In addition, control in the position and orientation of each surgical instrument can be achieved in the same principle as the control in the position and orientation of the flexible overtube body 10a.

Meanwhile, as illustrated in FIG. 2, the flexible overtube body 10a or the flexible surgical instruments 21 and 22 have a linear slope section according to the traction force of the traction wire to control the position and orientation of the surgical instrument. However, as illustrated in FIG. 2, even though the same traction force is provided when the surgical instrument is controlled by using the traction wire, there is a section in which the position and orientation are not uniformly controlled to correspond to the traction force. Such a section is referred to as a hysteresis region. In such a hysteresis region, even though the traction force is the same, the position and orientation control of the surgical instrument may be varied. The reason why such hysteresis occurs is as follows. When the flexible surgical instrument or the flexible overtube is inserted into the body, the shape or twisting of the flexible surgical instrument or the flexible overtube is changed, and it causes generation of friction force between a wire and a wires sheath or cause a wire elongation due to the use of the traction wire. Moreover, even if a control command is input to tow the wire due to a slack of the wire, a dead zone where there is no change in output may be formed. Furthermore, ununiform tube characteristics may be generated due to the sheath or wire in the flexible overtube.

Therefore, the present invention can compensatingly control the flexible surgical instrument having such hysteresis characteristics, thereby precisely controlling the surgical instrument.

(Configuration and function of hysteresis compensation control apparatus: First embodiment)

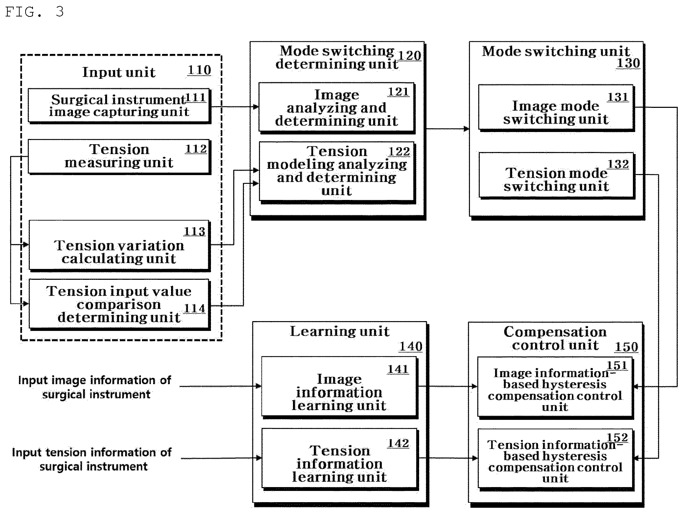

As illustrated in FIG. 3, the hysteresis compensation control apparatus of the flexible overtube according to a first embodiment of the present invention includes an input unit 110, a mode switching determining unit 120, a mode switching unit 130, a learning unit 140, and a compensation control unit 150.

The input unit 110 receives image information and tension information of a flexible surgical instrument required for a mode switching for a hysteresis control. Accordingly, the input unit 110 includes a surgical instrument image capturing unit 111, a tension measuring unit 112, a tension variation calculating unit 113, and a tension input value comparison determining unit 114.

The surgical instrument image capturing unit 111 is inserted into the third channel 13, and then, is inserted into the first channel 11 so as to generate an image obtained by capturing an end effector of the first surgical instrument 21 or a tip 21a of the surgical instrument, which is an object for hysteresis compensation control. In this instance, in a case in which the control object is the second surgical instrument 22, the surgical instrument image capturing unit 111 may capture the second surgical instrument 22, and if necessary, may capture both the first and second surgical instruments 21 and 22. On the other hand, the surgical instrument image capturing unit 111 may be fixed, or if necessary, may be inserted and fixed into the channel in a movement-controllable structure. In this instance, it is preferable that the capturing conditions of the camera inserted into the actual body for surgical operation exactly correspond to the capturing position, the capturing angle, or the capturing region of the surgical instrument which has been already utilized in the learning unit 140 for learning.

The tension measuring unit 112 measures the traction force of the traction wire of the surgical instrument. In order to measure the traction force, for instance, a tension measuring sensor for measuring the tension of the traction wire is disposed, or a load of a motor driving the traction wire disposed in the manipulation unit is measured.

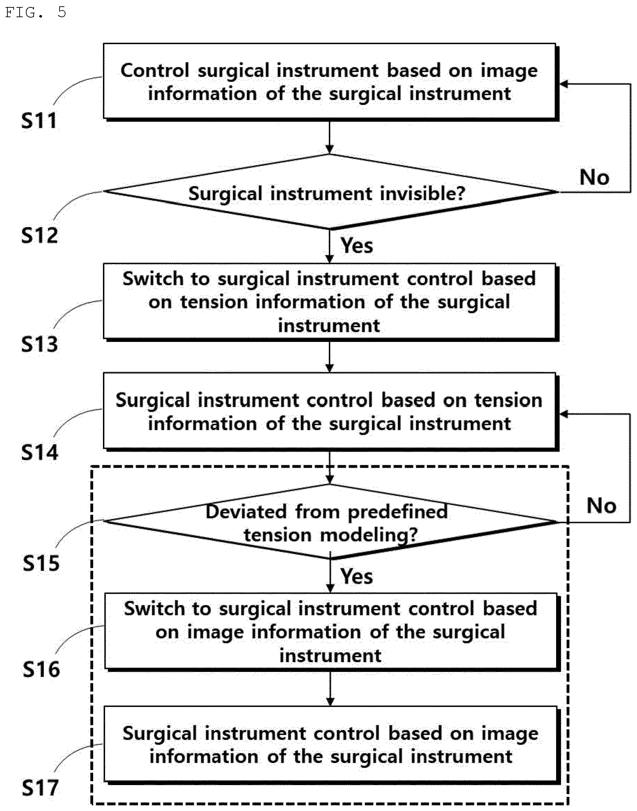

As illustrated in FIG. 5, after the control mode is switched from a surgical instrument hysteresis compensation control based on image information to a surgical instrument hysteresis compensation control based on tension information, in order to switch the control mode to the surgical instrument hysteresis compensation control based on image information, the hysteresis compensation control apparatus further includes a tension variation calculating unit 113 and a tension input value comparison determining unit 114.

The tension variation calculating unit 113 calculates a variation of a wire tension value when a load is applied to the first surgical instrument 21, namely, the hysteresis compensation control object, which has no load, or when a load applied to the first surgical instrument 21 is disappeared. In order to control any one of the surgical instruments with the traction wire, the two traction wires as illustrated in FIG. 1 must be respectively manipulated. So, a measuring sensor for measuring wire tension or traction force of the first and second traction wires is required.

For convenience in description, one traction wire for each surgical instrument is described, but in fact, two traction wires are required to move one degree of freedom, and four traction wires are required to move two degrees of freedom. In addition, one driving motor per one traction wire is required or one driving motor per two traction wires is required.

The tension variation calculating unit 113 calculates a variation of the tension values measured by the measuring sensors. Through comparison of the variation of the tension values, it is found whether to change from no load to load or to change from load to no load. In the two cases, since the tension information-based hysteresis compensation control is difficult, the control mode must be switched. That is, in the above cases, the control mode must be switched from tension information-based control to image information-based control.

The tension input value comparison determining unit 114 finds which any one among tension measuring values or traction force measuring values of the first and second wires is 'zero'. In a case in which any one among the tension values of the first and second wires is 'zero', since the tension information-based hysteresis compensation control is difficult, the control mode must be switched. That is, in the above cases, the control mode must be switched from tension information-based control to image information-based control. Hereinafter, assuming that the hysteresis compensation control object is the first surgical instrument 21, the present invention will be described. However, the hysteresis compensation control of the second surgical instrument 22 may be also described in the same principle.

The mode switching determining unit 120 according to an embodiment of the present invention determines the current state of the first surgical instrument 21 based on the image information of the first flexible surgical instrument 21 to generate a first control mode switching signal, which is a signal generated to switch the mode from the image information-based hysteresis compensation control to the tension information-based hysteresis compensation control.

The mode switching determining unit 120 determines whether the control mode corresponds a predefined tension control modeling on the basis of the tension information of the first surgical instrument 21 to generate a second control mode switching signal, which is a signal generated to switch the mode from the tension information-based hysteresis compensation control to the image information-based hysteresis compensation control.

The image information for determining the current state of the first surgical instrument 21 is image information obtained by capturing the tip 21a of the first surgical instrument 21.

The mode switching determining unit 120 includes an image analyzing and determining unit 121 and a tension modeling analyzing and determining unit 122.

The image analyzing and determining unit 121 receives an image of the tip area of the first surgical instrument 21 from the surgical instrument image capturing unit 111. The current state of the first surgical instrument 21 is determined through the received image. That is, in a case in which the tip area of the first surgical instrument 21 included in the image is not visible or the first surgical instrument 21 is hidden by the second surgical instrument 22, since the image information-based compensation control is impossible through the image analysis, the image analyzing and determining unit 121 generates a first control mode switching signal. The control mode is switched from the image information-based hysteresis compensation control to the tension information-based hysteresis compensation control according to the generated first control mode switching signal.

The tension modeling analyzing and determining unit 122 determines whether the control mode corresponds to the tension control modeling according to the input value of the tension variation calculating unit 113 or the tension input value comparison determining unit 114. When the tension modeling analyzing and determining unit 122 determines not to correspond to the tension control modeling, since the tension information-based compensation control is impossible any more, the tension modeling analyzing and determining unit 122 generates a second control mode switching signal. That is, the control mode is switched from the tension information-based hysteresis compensation control to the image information-based hysteresis compensation control. In this instance, for example, in a case in which a load of the first surgical instrument 21 is changed, namely, when a load is applied to the first surgical instrument 21, or when a load applied to the first surgical instrument 21 is disappeared, or in a case in which any one among the tension measuring values of the first and second traction wires towing the first surgical instrument 21 is 'zero', the cases do not correspond to the tension control modeling. The cases are not prediction models assumed for deep-learning of a tension information learning unit 142, which will be described later. In the above cases, the tension information-based hysteresis compensation control is impossible. Therefore, switching of the control mode is required.

The mode switching unit 130 according to an embodiment of the present invention includes an image mode switching unit 131 and a tension mode switching unit 132.

The image mode switching unit 131 switches the control mode from the image information-based compensation control of the first surgical instrument 21 to the tension information-based compensation control of the first surgical instrument 21 according to the first control mode switching signal of the image analyzing and determining unit 121.

The tension mode switching unit 132 switches the control mode from the image information-based compensation control of the first surgical instrument 21 to the tension information-based compensation control of the first surgical instrument 21 according to the second control mode switching signal of the tension modeling analyzing and determining unit 122.

The learning unit 140 according to an embodiment of the present invention performs deep learning or machine learning on the basis of image information or tension information, and calculates an image error value or a tension error value through learning. The learning unit 140 includes an image information learning unit 141 and a tension information learning unit 142.

The image information learning unit 141 performs machine learning or deep learning on the basis of the image of the tip 21a of the first surgical instrument 21. Therefore, it is preferable that the capturing conditions of the camera 23 for obtaining an image necessary for learning exactly correspond to the capturing conditions of the camera 23 when actually being inserted into a human body. The image information learning unit 141 calculates an image compensation control value or an image compensation error value corresponding to the image of the first surgical instrument 21 inserted into the body by learning according to a predefined learning model and captured now. The image compensation error value is transmitted to the compensation control unit 150. Of course, the generated image compensation error value may be information related to driving of the traction wire driving motor to actually drive the first surgical instrument.

The tension information learning unit 142 performs machine learning or deep learning based on the tension information of the traction wire of the first surgical instrument 21. The tension information learning unit 142 calculates a tension compensation control value or a tension compensation error value corresponding to the tension measuring value of the first surgical instrument 21 inserted into the body by learning according to the predefined learning model.

The compensation control unit 150 compensatingly controls the first surgical instrument 21 having a hysteresis curve by the image compensation error value or the tension compensation error value. The compensation control unit 150 includes an image information-based hysteresis compensation control unit 151 and a tension information-based hysteresis compensation control unit 152.

The image information-based hysteresis compensation control unit 151 performs a hysteresis compensation control of the first surgical instrument 21 using the image compensation error value. That is, the hysteresis compensation control is performed by the image information-based learning of the first surgical instrument captured currently.

The tension information-based hysteresis compensation control unit 152 performs a hysteresis compensation control of the first surgical instrument 21 using the tension compensation error value. That is, the hysteresis compensation control is performed by the tension information-based learning of the first surgical instrument captured currently.

### (Hysteresis compensation control method of flexible overtube)

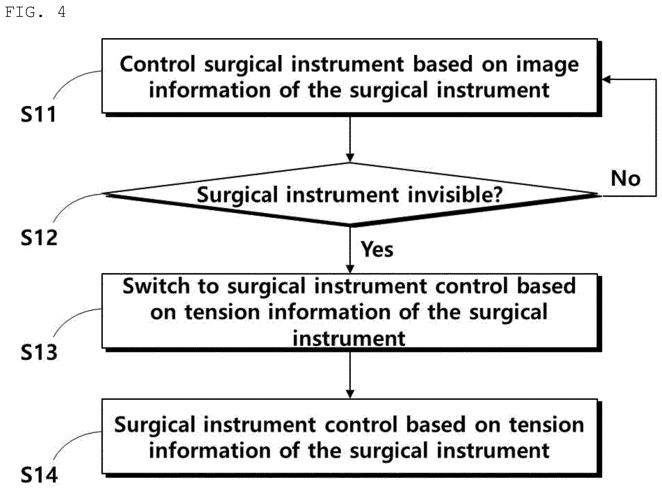

The hysteresis compensation control method of the flexible overtube according to an embodiment of the present invention includes a first method that the control mode is switched from the image information-based compensation control to the tension information-based compensation control as illustrated in FIG. 4, and a second method that the control mode is switched from the image information-based compensation control to the tension information-based compensation control and then is switched from the tension information-based compensation control to the image information-based compensation control. Hereinafter, the first method and the second method will be described.

As illustrated in FIG. 4, the method that the control mode is switched from the image information-based compensation control to the tension information-based compensation control is achieved through the following steps.

The compensation control unit 150 first compensatingly controls the first surgical instrument 21 having a hysteresis curve based on the image information of the first surgical instrument 21 (S11). More specifically, the image information-based hysteresis compensation control unit 151 controls tension of the traction wire of the first surgical instrument 21 based on the image information of the first surgical instrument 21.

In this instance, the image analyzing and determining unit 121 determines whether the first surgical instrument 21 is visible on the basis of the image information determining the current state of the first surgical instrument 21. In a case in which the first surgical instrument 21 is hidden or disappeared from a visible field of the camera unit 23, since the image information-based compensation control is impossible anymore, the image analyzing and determining unit 121 determines the current state of the first surgical instrument 21 (S12).

As a determination result, if it is determined that the surgical instrument is not visible, the mode switching unit 130 switches the control mode from the image information-based compensation control to the tension information-based compensation control (S13).

According to the switching of the control mode, the tension information-based hysteresis compensation control unit 152 compensatingly controls the first surgical instrument 21 having the hysteresis curve based on the tension information of the first surgical instrument 21.

Meanwhile, as illustrated in FIG. 5, after the control mode is switched from the image information-based compensation control to the tension information-based compensation control, the control mode is performed according to the following steps.

First, as illustrated in FIGS. 4 and 5, the steps from S11 to S14 are the same. The tension information-based hysteresis compensation control unit 152 compensatingly controls the first surgical instrument 21 based on the tension information of the first surgical instrument 21.

The tension modeling analyzing and determining unit 122 determines whether the control mode corresponds to the pre-defined tension control modeling based on the tension information of the first surgical instrument 21 after the control mode is switched from the image information-based compensation control to the tension information-based compensation control (S15). If the control mode does not correspond to the tension control modeling, the tension information-based compensation control cannot be performed anymore.

As a determination result, if it is determined that the control mode does not correspond to the predefined tension control modeling, the image mode switching unit 131 switches the control mode from the tension information-based compensation control to the image information-based compensation control (S16).

According to the switching of the control mode, the image information-based hysteresis compensation control unit 151 compensatingly controls the first surgical instrument 21 having the hysteresis curve based on the image information of the first surgical instrument 21.

### (Configuration and function of hysteresis compensation control apparatus: Second embodiment)

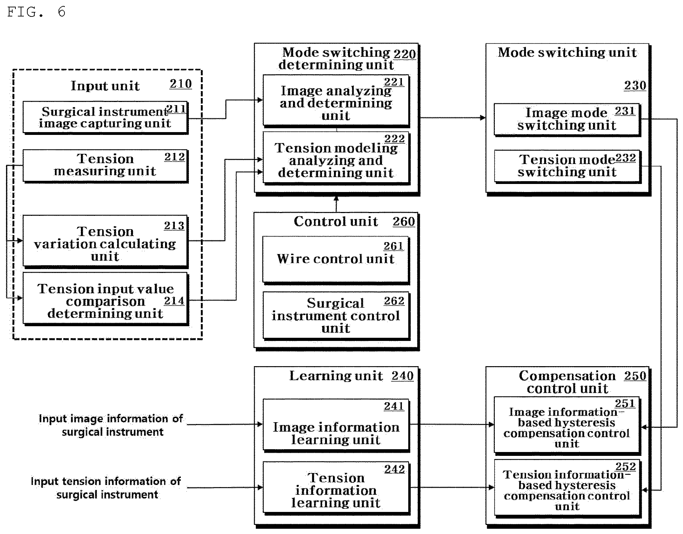

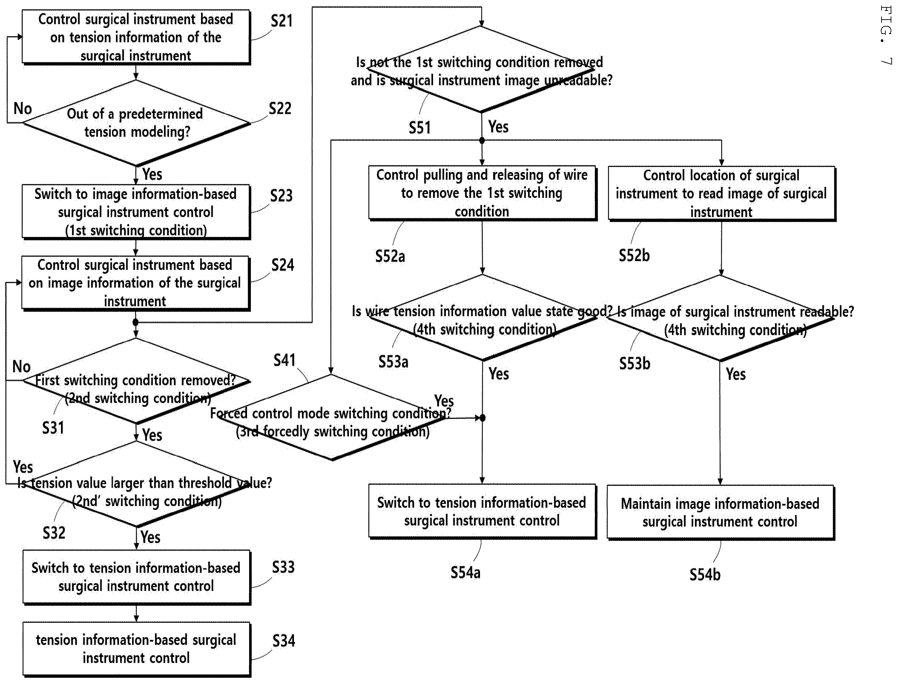

Referring to FIGS. 6 and 7, a hysteresis compensation control apparatus of the flexible overtube according to a second embodiment of the present invention will be described in detail. The hysteresis compensation control apparatus of the flexible overtube according to the second embodiment of the present invention may refer to descriptions of the input unit 110, the mode switching determining unit 120, the mode switching unit 130, the learning unit 140, and the compensation control unit 150 of the first embodiment if necessary, and the same descriptions will be omitted.

As illustrated in FIG. 6, an input unit 210 receives image information and tension information of a flexible surgical instrument required for a mode switching. The input unit 210 includes a surgical instrument image capturing unit 211, a tension measuring unit 212, a tension variation calculating unit 213, and a tension input value comparison determining unit 214. Description of the input unit 110 substitutes for the description of the input unit 210. However, the tension input value comparison determining unit 214 receives tension measuring values of first and second wires, and finds which any one among the tension measuring values is 'zero'. Accordingly, it is preferable to adjust traction force so that an initial tension measuring value according to the traction force of a wire is measured to be 'zero' or more even in no load.

A mode switching determining unit 220 includes an image analyzing and determining unit 221, and a tension modeling analyzing and determining unit 222. The mode switching determining unit 220 determines the current state of the surgical instrument based on the image information and the tension information of the flexible surgical instrument to generate control mode switching signals for switching control modes according to the current state of the surgical instrument. The control mode switching signals are respectively generated according to switching conditions. The mode switching determining unit 220 generates first and second switching condition control mode switching signals, a third forced switching condition control mode switching signal, and a signal for inducing a state change of the surgical instrument.

The tension modeling analyzing and determining unit 222 generates the first switching condition control mode switching signal, which switches the control mode from a tension information-based flexible tube control to an image information-based flexible tube control under the first switching condition determining that it is impossible to perform the tension information-based flexible tube control while performing a tension information-based control. The above description of the tension information-based control substitutes for an example of the first switching condition.

Furthermore, the tension modeling analyzing and determining unit 222 generates the second switching condition control mode switching signal, which switches the control mode from an image information-based flexible tube control into a tension information-based flexible tube control, under the second switching condition determining that it is impossible to perform the image information-based flexible tube control according to the signal of the image analyzing and determining unit 221 and determining that the first switching condition was removed, while performing the image information-based flexible tube control by the first switching condition control mode switching signal.

The image analyzing and determining unit 221 generates the third forced switching condition control mode switching signal which forcedly switches the control mode from the image information-based flexible tube control into the tension information-based flexible tube control, under the third forced switching condition determining that it is impossible to perform the image information-based flexible tube control and determining that the first switching condition was not removed, while performing the image information-based flexible tube control by the first switching condition control mode switching signal. As examples of the third forced switching condition, there are several cases in which a portion of at least one among the plurality of surgical instruments is not shown on an image, in which two or more surgical instruments are overlapped each other so that it is difficult to read the image due to an overlapped zone formed on the image, and in which it is difficult to read the image do to two or more surgical instruments getting in contact with each other. As other examples, there are several cases in which the visible field of the camera or the surgical instrument is blocked by the blood stained on a camera lens or the surgical instrument or by the organs in a human body or by smoke caused by resection of the bowel, in which it is difficult to grasp the surgical instrument since the entire image outline of the surgical instrument is changed by the organs or by the environment around the organs, and in which it is difficult to identify the image of the surgical instrument since LED illumination of the camera is reflected to the surgical instrument when any one among the surgical instruments hides the visible field of the camera.

The mode switching determining unit 220 generates the signal inducing a state change of the surgical instrument to make the tension information-based or the image information-based flexible tube control possible by controlling the wire or the surgical instrument, under the fourth switching condition determining that it is impossible to perform the image information-based flexible tube control and determining that the first switching condition was not removed, while performing the image information-based flexible tube control by the first switching condition control mode switching signal. The generated signal is transferred to a control unit 260 which will be described later.

The control unit 260 includes a wire control unit 261 and a surgical instrument control unit 262. The wire control unit 261 controls wires to control the tension information-based flexible tube by controlling the wires after receiving the signal inducing the state change of the surgical instrument from the mode switching determining unit 220. As an example, the control unit repeatedly controls the wire to be pulled or released so that a tension measurement value is measured if the wire is not broken.

Furthermore, the surgical instrument control unit 262 receives the signal inducing the state change of the surgical instrument from the mode switching determining unit 220 to control the surgical instrument image capturing unit 211 or the surgical instrument, so that it is possible to control the flexible tube based on the image-readable image information.

In a case in which the tension measurement value is input normally or is image-readable by the control of the control unit 260, the control unit generates a possible tension information signal and a possible image information signal, and transmits them to the mode switching determining unit 220.

The mode switching determining unit 220 generates a tension state change control mode switching signal, which switches the control mode from the image information-based flexible tube control into the tension information-based flexible tube control when the possible tension information signal is received from the control unit 260. The mode switching determining unit 220 is continuously controlled in the image information-based flexible tube control mode when receiving the possible image information signal from the control unit 260. Therefore, there is no need to switch the control mode.

If there is no state change even by the control of the control unit 260 and it is impossible to control based on the tension information and the image information, the mode switching determining unit 220 sends feedback to a user.

A mode switching unit 230 receives the first and second switching condition control mode switching signals, the third forced switching condition control mode switching signal, and the tension state change control mode switching signal from the mode switching determining unit 220. The mode switching unit 230 controls to switch the control mode from the tension information-based flexible tube control to the image information-based flexible tube control or from the image information-based flexible tube control to the tension information-based flexible tube control according to the control mode switching signals of the mode switching determining unit 220.

The above descriptions substitute for descriptions of a learning unit 240 and a compensation control unit 250.

As illustrated in FIG. 7, the tension information-based flexible tube control is first performed (S21). In a case in which it is determined not to correspond to the predefined tension control modeling (first switching condition) while performing the tension information-based flexible tube control (S22), it is controlled to change the control mode into the image information-based flexible tube control (S23 and S24).

Meanwhile, while controlling from the tension information-based flexible tube control to the image information-based flexible tube control, in a case in which the first switching condition is removed (second switching condition) or the first switching condition is not removed and it is impossible to read the image of the surgical instrument so that the image information-based flexible tube control cannot be maintained anymore, the control is performed as follows.

First, in a case in which the first switching condition is removed (second switching condition) and the tension measurement value is larger than a predetermined threshold value, for instance, the threshold value is 'zero', (second switching condition), the control mode is switched from the image information-based flexible tube control to the tension information-based flexible tube control (S31, S32, S33, and S34).

Meanwhile, in a case in which the first switching condition is not removed and it is impossible to read the image of the surgical instrument (S51), control is performed by the following three methods.

First, as the first method, it is determined whether to conform to the forced control mode switching condition (third forced switching condition), and if it conforms to the forced control mode switching condition, the control mode is unconditionally switched from the image information-based flexible tube control to the tension information-based flexible tube control (S41 and S54a) .

As the second method, the wire is controlled such that the first switching condition is released by repeatedly controlling the wire so as to be pulled or released (S52a). In a case in which the tension measurement value of the wire is input normally according to control of the wire (fourth switching condition), the control mode is unconditionally switched from the image information-based flexible tube control to the tension information-based flexible tube control (S53a and S54a). In a case in which the tension measurement value is not input normally not to satisfy the fourth switching condition, the second method is changed to the following third method or feedback is sent to the user finally.

As the third method, the position of the surgical instrument or the surgical instrument image capturing unit 211 is controlled so that the image of the surgical instrument is readable (S52b). In a case in which it is determined that it is possible to read the image according to the control of location (fourth switching condition), the image information-based flexible tube control is maintained (S53b and S54b). If it is impossible to read the image not to satisfy the fourth switching condition, the third method is changed to the above-mentioned second method or feedback is sent to the user finally.

### (Configuration and function of hysteresis compensation control apparatus: Third embodiment, not forming part of the claimed invention)

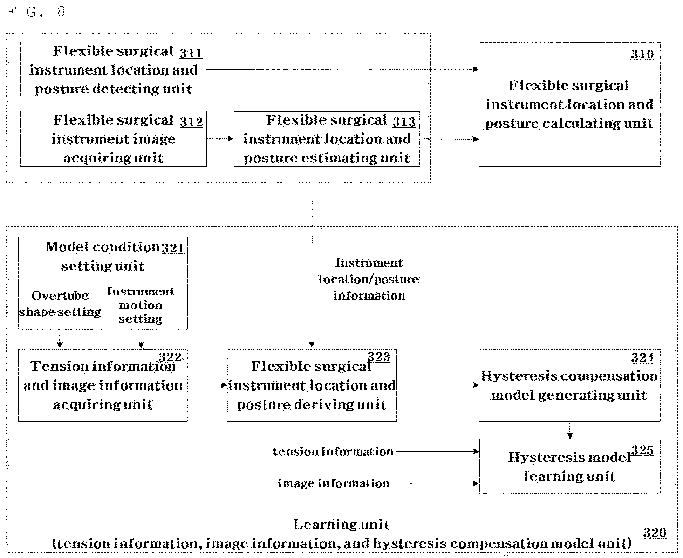

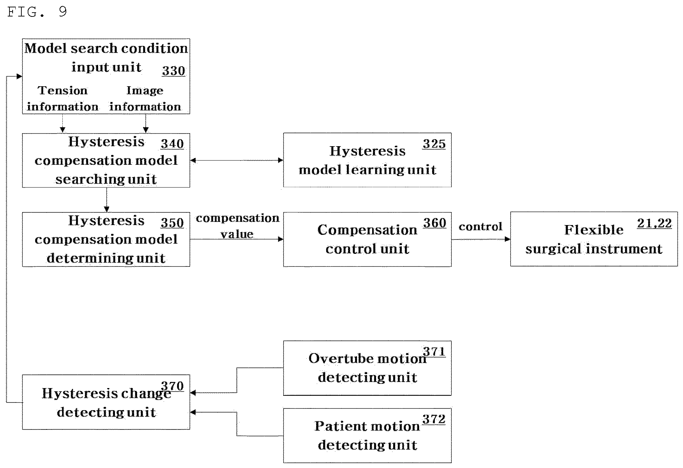

In general, hysteresis is varied according to the level of friction between a wire and a sheath, and the shape of the sheath is also varied according to the shape of an overtube. Therefore, friction between the wire and the sheath is changed. So, characteristics of the hysteresis are changed, and so, the position and posture (bending angle) of the flexible surgical instrument are changed according to the shape of the overtube even if there is driving force of the same wires. Therefore, the hysteresis compensation control apparatus for the flexible overtube according to the third embodiment of the present invention is an apparatus to search a hysteresis characteristics model suitable for the change in shape of the overtube and changes in location and posture of the flexible surgical instrument to derive a compensation value. Referring to FIGS. 8 and 9, the hysteresis compensation control apparatus for the flexible overtube according to the third embodiment of the present invention will be described in detail.

First, the hysteresis compensation model is learned and corrected through learning of the hysteresis model, the corrected hysteresis compensation model is searched according to model searching conditions to determine a hysteresis compensation model. A compensation value is derived by the determined compensation model, thereby compensating and controlling the flexible surgical instruments 21 and 22.

First, referring to FIG. 8, learning of the hysteresis model will be described. As illustrated in FIG. 8, a flexible surgical instrument location and posture calculating unit 310 calculates location and posture values of the flexible surgical instruments 21 and 22 driven by traction of the wire while being inserted into the channel of the overtube. The location and posture value of the flexible surgical instrument may be calculated by the following two methods.

In the first method, a flexible surgical instrument location and posture detecting unit 311 detects and calculates an actual location and posture value of the flexible surgical instrument through sensors. So, various sensors are arranged. As an example of the sensors, there are FBG sensors (optical fiber grating elements), magnetic sensors, or cameras. The actual location and posture value of the flexible surgical instrument may be a bending angle of the flexible surgical instrument or a measurement value according to forward and backward movement or rotation of the flexible surgical instrument. The tension information may be a tension value or a pulled amount of the wire measured with an encoder.

In the second method, a flexible surgical instrument image acquiring unit 312 acquires an image of the flexible surgical instrument. The acquired image of the flexible surgical instrument is stored as a data set. A flexible surgical instrument location and posture estimating unit 313 matches a location and posture value of the flexible surgical instrument with an image data set of the input flexible surgical instrument and stores it as a data set. Therefore, the flexible surgical instrument location and posture estimating unit 313 can estimate an actual location and posture value of the flexible surgical instrument matched based on the image data set transmitted from the flexible surgical instrument image acquiring unit 312. The image information of the flexible surgical instrument is the image data set displayed on a camera 111 inserted into a channel of an overtube.

A model condition setting unit 321 sets to control a shape of the overtube into a predetermined shape or to control a motion of the flexible surgical instrument into a predetermined motion in order to set a hysteresis compensation model condition. As an example, the overtube can be changed into various shapes, and also the motion of the flexible surgical instrument can be varied according to surgical operations. Therefore, the model condition setting unit 321 sets various possible environment variables (shape of the overtube and motion of the flexible surgical instrument), and generates a hysteresis compensation model according to the environment variable setting.

A tension information and image information acquiring unit 322 acquires tension information and image information according to hysteresis compensation model conditions set by the model condition setting unit 321 in various ways. Additionally, the tension information and image information acquiring unit 322 stores an input data set (input information) for inputting the acquired tension information and image information into a hysteresis compensation model generating unit. The tension information can be measured through the tension measuring unit 112, and the image information can be acquired through the flexible surgical instrument image acquiring unit 312 or the surgical instrument image capturing unit 111.

A flexible surgical instrument location and posture deriving unit 323 derives location and posture values of the flexible surgical instrument when the tension information and image information acquiring unit 322 obtains input information. As described above, the location and posture value of the flexible surgical instrument can be derived from the sensor or the image data set. Additionally, the flexible surgical instrument location and posture deriving unit 323 stores the derived location and posture value of the flexible surgical instrument as an output data set (output information).

A hysteresis compensation model generating unit 324 receives tension information and image information of the wire, which are the input data set obtained according to the shape control of the overtube and the motion control of the flexible surgical instrument set by the model condition setting unit 321, and the location and posture value of the flexible surgical instrument, which is the output data set when the input information is obtained. The hysteresis compensation model generating unit 324 receives the input data set and the output data set to generate hysteresis compensation models according to various environment variables set by the model condition setting unit 321, namely, according to a change in shape of the overtube and a change in motion of the flexible surgical instrument.

A hysteresis model learning unit 325 learns the tension information, the image information, and the hysteresis compensation model generated by the hysteresis compensation generating unit 324 to calibrate the hysteresis compensation model.

As described above, after learning and calibrating the hysteresis compensation model through the hysteresis model learning, the hysteresis model learning unit 325 searches the calibrated hysteresis compensation model according to model search conditions to determine a hysteresis compensation model, and derives a compensation value by the determined compensation model to compensate and control the flexible surgical instruments 21 and 22. Hereinafter, referring to FIG. 9, the hysteresis compensation control apparatus according to the third embodiment of the present invention will be described.

A model search condition input unit 330 obtains tension information and image information caused by a specific motion of the flexible surgical instrument after the overtube reaches a target position having a lesion. As described above, the tension information can be measured by the tension measuring unit 112, and the image information can be obtained through the flexible surgical instrument image obtaining unit 312 or the surgical instrument image capturing unit 111. The obtained tension information and image information is transmitted to a hysteresis compensation model searching unit 340.

The hysteresis compensation model searching unit 340 searches the calibrated hysteresis compensation model of the hysteresis model learning unit 325 based on the tension information and the image information input by the model search condition input unit 330.

A hysteresis compensation model determining unit 350 determines the calibrated hysteresis compensation model searched by the hysteresis compensation model searching unit 340, and calculates a compensation value, which is a tension error value, by the determined hysteresis compensation model.

A compensation control unit 360 compensates and controls the flexible tube having the hysteresis characteristics on the basis of the tension error value of the hysteresis compensation model determined by the hysteresis compensation model determining unit 350.

A hysteresis change detecting unit 370 detects motions of the overtube or motions of a patient. The motions of the overtube or the patient correspond to the hysteresis change conditions. In a case in which there is a hysteresis change, it may be necessary to re-search the determined hysteresis compensation model. Therefore, the hysteresis change detecting unit 370 transmits a detection signal to the model search condition input unit 330 when detecting the hysteresis change condition. The model search condition input unit 330 receiving the hysteresis change detection signal re-obtains tension information and image information to re-search the hysteresis compensation model.

The motion of the overtube may be detected, for instance, in a case in which the image is changed without a control input to control the overtube or the flexible surgical instrument, or in a case in which a master manipulation signal to control the overtube is input.

Meanwhile, if the patient moves, the shape of the overtube inserted into the patient's body may be changed. Therefore, the hysteresis change detecting unit 370 predicts the hysteresis change by detecting motions of the patient. The hysteresis change detecting unit 370 can detect the patient's motion by checking the patient's breathing or through changes in images of the patient.

## Claims

1. A hysteresis compensation control apparatus for a flexible overtube (10), comprising:
an input unit (110, 210) receiving image information and tension information of a flexible surgical instrument required for mode switching;
a mode switching determining unit (120, 220) for determining a current state of the surgical instrument on the basis of the image information and the tension information of the flexible surgical instrument, and generating a control mode switching signal for switching a control mode according to the current state of the surgical instrument;
a mode switching unit (130, 230) configured for switching a control mode from a tension information-based flexible tube control to an image information-based flexible tube control and for switching a control mode from an image information-based flexible tube control to a tension information-based flexible tube control according to the control mode switching signal; and
a compensation control unit (150, 250) for compensatingly controlling a flexible surgical instrument having hysteresis characteristics on the basis of a tension error value calculated by a learning model.

2. The hysteresis compensation control apparatus according to claim 1, wherein the input unit (110, 210) includes:
a surgical instrument image capturing unit (111, 211) inserted into the channel of the tube body to capture an image of the surgical instrument;
a tension measuring unit (112, 212) for measuring traction force of a traction wire (10b, 10c) of the surgical instrument;
a tension variation calculating unit (113, 213) calculating a tension variation that a wire tension value of the wire (10b, 10c) is varied on the basis of traction force measured by the tension measuring unit (112, 212); and
a tension input value comparison determining unit (114, 214) comparing and determining the wire tension value input on the basis of traction force measured by the tension measuring unit (112, 212).

3. The hysteresis compensation control apparatus according to claim 1, wherein the mode switching determining unit (120, 220):
generates a first switching condition control mode switching signal, which switches the control mode from a tension information-based flexible tube control to an image information-based flexible tube control under a first switching condition determining that it is impossible to perform the tension information-based flexible tube control; or
generates a second switching condition control mode switching signal, which switches the control mode from an image information-based flexible tube control to a tension information-based flexible tube control under a second switching condition determining that it is impossible to perform the image information-based flexible tube control and the first switching condition is removed while performing the image information-based flexible tube control by the first switching condition control mode switching signal; or
generates a third switching condition control mode switching signal, which forcedly switches the control mode from an image information-based flexible tube control to a tension information-based flexible tube control under a third forcedly switching condition determining that it is impossible to perform the image information-based flexible tube control and the first switching condition is not removed while performing the image information-based flexible tube control by the first switching condition control mode switching signal; or
generates a signal for inducing a state change of the surgical instrument to enable the tension information or image information-based flexible tube control by controlling the wire or the surgical instrument under a fourth switching condition determining that it is impossible to perform the image information-based flexible tube control and the first switching condition is not removed while performing the image information-based flexible tube control by the first switching condition control mode switching signal.

4. The hysteresis compensation control apparatus according to claim 3, further comprising:
a control unit (260) for controlling a wire (10b, 10c) by receiving the signal for inducing a state change of the surgical instrument to enable the tension information-based flexible tube control or for controlling any one among the surgical instrument image capturing unit (211) and the surgical instrument to enable the image information-based flexible tube control.

5. The hysteresis compensation control apparatus according to claim 1, further comprising:
an image information learning unit (141, 241) for learning according to a predetermined learning model based on image information of the surgical instrument and calculating an image error value by the learning; and
a tension information learning unit (142, 242) for learning according to a predetermined learning model based on tension information of the surgical instrument and calculating a tension error value by the learning.

6. The hysteresis compensation control apparatus according to claim 1, wherein the mode switching unit (120, 220) switches an image information-based flexible tube control to a tension information-based flexible tube control according to the control mode switching signal of the mode switching determining unit (120, 220).

7. The hysteresis compensation control apparatus according to claim 6, wherein the image information for determining a current state of the surgical instrument is image information obtained by capturing a tip region of the surgical instrument inserted into a channel of a tube body and towed by a traction wire.

8. The hysteresis compensation control apparatus according to claim 7, wherein the input unit (110, 120) includes:
a surgical instrument image capturing unit (111, 211) inserted into the channel of the tube body to capture an image of the surgical instrument; and
a tension measuring unit (112, 212) for measuring traction force of a traction wire of the surgical instrument,
wherein the image information of the tip region of the surgical instrument captured by the surgical instrument image capturing unit (111, 211) is transmitted to the mode switching determining unit (120, 220) to determine the current state of the surgical instrument.

9. The hysteresis compensation control apparatus according to claim 8, wherein the mode switching determining unit (120, 220) determines whether it is possible to control the image information-based flexible tube control mode on the basis of the image information of the tip region of the surgical instrument.

## Patentansprüche

1. Hysteresekompensationssteuerungsvorrichtung für einen flexiblen Übertubus (10), umfassend:
eine Eingabeeinheit (110, 210), die Bildinformationen und Zugspannungsinformationen eines flexiblen chirurgischen Instruments empfängt, die für eine Modusumschaltung erforderlich sind;
eine Modusumschalt-Bestimmungseinheit (120, 220) zum Bestimmen eines momentanen Zustands des chirurgischen Instruments auf der Grundlage der Bildinformationen und der Zugspannungsinformationen des flexiblen chirurgischen Instruments und Generieren eines Steuerungsmodus-Umschaltsignals zum Umschalten eines Steuerungsmodus gemäß dem momentanen Zustand des chirurgischen Instruments;
eine Modusumschalteinheit (130, 230), die dafür ausgebildet ist, einen Steuerungsmodus von einer zugspannungsinformationsbasierten Steuerung des flexiblen Tubus zu einer bildinformationsbasierten Steuerung des flexiblen Tubus umzuschalten und einen Steuerungsmodus von einer bildinformationsbasierten Steuerung des flexiblen Tubus zu einer zugspannungsinformationsbasierten Steuerung des flexiblen Tubus gemäß dem Steuerungsmodus-Umschaltsignal umzuschalten; und
eine Kompensationssteuereinheit (150, 250) zum kompensierenden Steuern eines flexiblen chirurgischen Instruments, das Hysterese-Eigenschaften aufweist, auf der Grundlage eines durch ein Lernmodell berechneten Zugspannungsfehlerwertes.

2. Hysteresekompensationssteuerungsvorrichtung nach Anspruch 1, wobei die Eingabeeinheit (110, 210) aufweist:
eine Bildaufnahmeeinheit (111, 211) für chirurgische Instrumente, die in den Kanal des Tubuskörpers eingeführt ist, um ein Bild des chirurgischen Instruments aufzunehmen;
eine Zugspannungsmesseinheit (112, 212) zum Messen einer Zugkraft eines Zugdrahtes (10b, 10c) des chirurgischen Instruments;
eine Zugspannungsvariations-Berechnungseinheit (113, 213), die eine Zugspannungsvariation, um die ein Zugspannungswert des Drahtes (10b, 10c) variiert wird, auf der Grundlage einer durch die Zugspannungsmesseinheit (112, 212) gemessenen Zugkraft berechnet; und
eine Zugspannungseingabewert-Vergleichsbestimmungseinheit (114, 214), die den eingegebenen Drahtzugspannungswert auf der Grundlage einer durch die Zugspannungsmesseinheit (112, 212) gemessenen Zugkraft vergleicht und bestimmt.

3. Hysteresekompensationssteuerungsvorrichtung nach Anspruch 1, wobei die Modusumschalt-Bestimmungseinheit (120, 220):
ein Steuerungsmodus-Umschaltsignal für eine erste Umschaltbedingung generiert, das den Steuerungsmodus von einer zugspannungsinformationsbasierten Steuerung des flexiblen Tubus zu einer bildinformationsbasierten Steuerung des flexiblen Tubus unter einer ersten Umschaltbedingung umschaltet, die bestimmt, dass es unmöglich ist, die zugspannungsinformationsbasierte Steuerung des flexiblen Tubus durchzuführen; oder
ein Steuerungsmodus-Umschaltsignal für eine zweite Umschaltbedingung generiert, das den Steuerungsmodus von einer bildinformationsbasierten Steuerung des flexiblen Tubus zu einer zugspannungsinformationsbasierten Steuerung des flexiblen Tubus unter einer zweiten Umschaltbedingung umschaltet, die bestimmt, dass es unmöglich ist, die bildinformationsbasierte Steuerung des flexiblen Tubus durchzuführen, und die erste Umschaltbedingung aufgehoben ist, während die bildinformationsbasierte Steuerung des flexiblen Tubus durch das Steuerungsmodus-Umschaltsignal für eine erste Umschaltbedingung durchgeführt wird; oder
ein Steuerungsmodus-Umschaltsignal für eine dritte Umschaltbedingung generiert, das den Steuerungsmodus zwangsweise von einer bildinformationsbasierten Steuerung des flexiblen Tubus zu einer zugspannungsinformationsbasierten Steuerung des flexiblen Tubus unter einer dritten Zwangsumschaltbedingung umschaltet, die bestimmt, dass es unmöglich ist, die bildinformationsbasierte Steuerung des flexiblen Tubus durchzuführen, und die erste Umschaltbedingung nicht aufgehoben ist, während die bildinformationsbasierte Steuerung des flexiblen Tubus durch das Steuerungsmodus-Umschaltsignal für die erste Umschaltbedingung durchgeführt wird; oder
ein Signal generiert, um eine Zustandsänderung des chirurgischen Instruments zu induzieren, um die zugspannungsinformations- oder bildinformationsbasierte Steuerung des flexiblen Tubus durch Steuern des Drahtes oder des chirurgischen Instruments unter einer vierten Umschaltbedingung zu ermöglichen, die bestimmt, dass es unmöglich ist, die bildinformationsbasierte Steuerung des flexiblen Tubus durchzuführen und die erste Umschaltbedingung nicht aufgehoben ist, während die bildinformationsbasierte Steuerung des flexiblen Tubus durch das Steuerungsmodus-Umschaltsignal für eine erste Umschaltbedingung durchgeführt wird.

4. Hysteresekompensationssteuerungsvorrichtung nach Anspruch 3, des Weiteren umfassend:
eine Steuereinheit (260) zum Steuern eines Drahtes (10b, 10c) durch Empfangen des Signals zum Induzieren einer Zustandsänderung des chirurgischen Instruments, um die zugspannungsinformationsbasierte Steuerung des flexiblen Tubus zu ermöglichen, oder zum Steuern einer der Bildaufnahmeeinheit (211) für chirurgische Instrumente und des chirurgischen Instruments, um die bildinformationsbasierte Steuerung des flexiblen Tubus zu ermöglichen.

5. Hysteresekompensationssteuerungsvorrichtung nach Anspruch 1, des Weiteren umfassend:
eine Bildinformations-Lerneinheit (141, 241) zum Lernen gemäß einem zuvor festgelegten Lernmodell auf der Grundlage von Bildinformationen des chirurgischen Instruments und zum Berechnen eines Bildfehlerwertes durch das Lernen; und
eine Zugspannungsinformations-Lerneinheit (142, 242) zum Lernen gemäß einem zuvor festgelegten Lernmodell auf der Grundlage von Zugspannungsinformationen des chirurgischen Instruments und Berechnen eines Zugspannungsfehlerwertes durch das Lernen.

6. Hysteresekompensationssteuerungsvorrichtung nach Anspruch 1, wobei die Modusumschalteinheit (120, 220) gemäß dem Steuerungsmodus-Umschaltsignal der Modusumschalt-Bestimmungseinheit (120, 220) eine bildinformationsbasierte Steuerung des flexiblen Tubus zu einer zugspannungsinformationsbasierten Steuerung des flexiblen Tubus umschaltet.

7. Hysteresekompensationssteuerungsvorrichtung nach Anspruch 6, wobei die Bildinformationen zum Bestimmen eines momentanen Zustands des chirurgischen Instruments Bildinformationen sind, die durch Aufnehmen einer Spitzenregion des chirurgischen Instruments erlangt werden, das in einen Kanal eines Tubuskörpers eingeführt und von einem Zugdraht gezogen wird.

8. Hysteresekompensationssteuerungsvorrichtung nach Anspruch 7, wobei die Eingabeeinheit (110, 120) aufweist:
eine Bildaufnahmeeinheit (111, 211) für chirurgische Instrumente, die in den Kanal des Tubuskörpers eingeführt ist, um ein Bild des chirurgischen Instruments aufzunehmen; und
eine Zugspannungsmesseinheit (112, 212) zum Messen einer Zugkraft eines Zugdrahtes des chirurgischen Instruments,
wobei die durch die Bildaufnahmeeinheit (111, 211) für chirurgische Instrumente aufgenommenen Bildinformationen der Spitzenregion des chirurgischen Instruments an die Modusumschalt-Bestimmungseinheit (120, 220) gesendet werden, um den momentanen Zustand des chirurgischen Instruments zu bestimmen.

9. Hysteresekompensationssteuerungsvorrichtung nach Anspruch 8, wobei die Modusumschalt-Bestimmungseinheit (120, 220) auf der Grundlage der Bildinformationen der Spitzenregion des chirurgischen Instruments bestimmt, ob es möglich ist, den Modus der bildinformationsbasierten Steuerung des flexiblen Tubus zu steuern.

## Revendications

1. Un appareil de commande de compensation d'hystérésis pour un surtube flexible (10), comprenant :
une unité d'entrée (110, 210), recevant des informations d'image et des informations de tension d'un instrument chirurgical flexible nécessaires à une commutation de mode ;
une unité (120, 220) de détermination de changement de mode, destinée à déterminer l'état actuel de l'instrument chirurgical sur la base des informations d'image et des informations de tension de l'instrument chirurgical flexible, et à générer un signal de changement de mode de commande pour changer de mode de commande en fonction de l'état actuel de l'instrument chirurgical ;
une unité (130, 230) de commutation de mode, destinée à passer d'un mode de commande basé sur les informations de tension à une commande de tube flexible basée sur les informations d'image, et à passer d'un mode de commande basé sur les informations d'image à une commande de tube flexible basée sur les informations de tension, en fonction du signal de commutation de mode de commande ; et
une unité (150, 250) de commande de compensation, destinée à commander de manière compensée un instrument chirurgical flexible présentant des caractéristiques d'hystérésis sur la base d'une valeur d'erreur de tension calculée par un modèle d'apprentissage.

2. L'appareil de commande de compensation d'hystérésis selon la revendication 1, dans lequel l'unité d'entrée (110, 210) comprend :
une unité (111, 211) de capture d'image de l'instrument chirurgical, insérée dans le canal du corps du tube de manière à capturer une image de l'instrument chirurgical ;
une unité (112, 212) de mesure de tension, destinée à mesurer la force de traction d'un fil de traction (10b, 10c) de l'instrument chirurgical ;
une unité (113, 213) de calcul de variation de tension, calculant une variation de tension selon laquelle la valeur de tension d'un fil (10b, 10c) varie en fonction de la force de traction mesurée par l'unité (112, 212) de mesure de tension ; et
une unité (114, 214) de détermination de comparaison de la valeur de tension d'entrée, qui compare et détermine la valeur entrée de tension du fil (10b, 10c) en fonction de la force de traction mesurée par l'unité (112, 212) de mesure de tension.

3. L'appareil de commande de compensation d'hystérésis selon la revendication 1, dans lequel l'unité (120, 220) de détermination de changement de mode :
génère un premier signal de commutation du mode de commande selon une condition de commutation, qui fait passer le mode de commande d'une commande du tube flexible basée sur les informations de tension à une commande du tube flexible basée sur les informations d'image selon une première condition de commutation déterminant qu'il est impossible d'effectuer la commande de tube flexible basée sur les informations de tension ; ou
génère un deuxième signal de commutation du mode de commande selon une condition de commutation, qui fait passer le mode de commande d'une commande du tube flexible basée sur des informations d'image à une commande du tube flexible basée sur des informations de tension selon une deuxième condition de commutation déterminant qu'il est impossible d'effectuer la commande du tube flexible basée sur des informations d'image et que la première condition de commutation a été levée pendant l'exécution de la commande du tube flexible basée sur des informations d'image, par le premier signal de commutation de mode de commande ; ou
génère un troisième signal de commutation du mode de commande selon une condition de commutation, qui fait passer de manière forcée le mode de commande d'une commande du tube flexible basée sur les informations d'image à une commande du tube flexible basée sur les informations de tension selon une troisième condition de commutation forcée déterminant qu'il est impossible d'effectuer la commande du tube flexible basée sur des informations d'image et que la première condition de commutation n'est pas levée pendant l'exécution de la commande du tube flexible basée sur les informations d'image, par le premier signal de commutation de mode de commande selon une condition de commutation ; ou
génère un signal destiné à induire un changement d'état de l'instrument chirurgical afin de permettre la commande du tube flexible basée sur les informations de tension ou sur les informations d'image, en commandant le fil (10b, 10c) ou l'instrument chirurgical selon une quatrième condition de commutation déterminant qu'il est impossible d'effectuer la commande du tube flexible basée sur les informations d'image et que la première condition de commutation n'est pas levée pendant l'exécution de la commande du tube flexible basée sur les informations d'image par le signal de commutation de mode de commande selon la première condition de commutation.

4. L'appareil de commande de compensation d'hystérésis selon la revendication 3, comprenant en outre :
une unité de commande (260), destinée à commander le fil (10b, 10c) en recevant le signal destiné à induire un changement d'état de l'instrument chirurgical afin de permettre la commande du tube flexible basée sur les informations de tension, ou à commander l'un quelconque parmi l'unité (211) de capture d'images de l'instrument chirurgical et l'instrument chirurgical afin de permettre la commande du tube flexible basée sur les informations d'image.

5. L'appareil de commande de compensation d'hystérésis selon la revendication 1, comprenant en outre :
une unité (141, 241) d'apprentissage des informations d'image, destinée à effectuer un apprentissage selon un modèle d'apprentissage prédéterminé sur la base d'informations d'image de l'instrument chirurgical, et à calculer une valeur d'erreur d'image à l'issue de cet apprentissage ; et
une unité (142, 242) d'apprentissage des informations de tension, destinée à effectuer un apprentissage selon un modèle d'apprentissage prédéterminé sur la base d'informations de tension de l'instrument chirurgical, et à calculer une valeur d'erreur de tension à l'issue de cet apprentissage.

6. L'appareil de commande de compensation d'hystérésis selon la revendication 1, dans lequel l'unité (120, 220) de commutation de mode fait passer d'une commande du tube flexible basée sur les informations d'image à une commande du tube flexible basée sur les informations de tension, conformément au signal de commutation de mode de l'unité (120, 220) de détermination de changement de mode.

7. L'appareil de commande de compensation d'hystérésis selon la revendication 6, dans lequel les informations d'image destinées à déterminer un état actuel de l'instrument chirurgical sont des informations d'image obtenues en capturant une zone d'extrémité de l'instrument chirurgical inséré dans un canal d'un corps de tube et tiré par un fil de traction.

8. L'appareil de commande de compensation d'hystérésis selon la revendication 7, dans lequel l'unité d'entrée (110, 210) comprend :
une unité (111, 211) de capture d'image de l'instrument chirurgical, insérée dans le canal du corps de tube pour capturer une image de l'instrument chirurgical ; et
une unité (112, 212) de mesure de tension destinée à mesurer la force de traction d'un fil de traction de l'instrument chirurgical,
dans lequel les informations d'image de la zone d'extrémité de l'instrument chirurgical capturées par l'unité (111, 211) de capture d'images de l'instrument chirurgical sont transmises à l'unité (120, 220) de détermination de changement de mode afin de déterminer l'état actuel de l'instrument chirurgical.

9. L'appareil de commande de compensation d'hystérésis selon la revendication 8, dans lequel l'unité (120, 220) de détermination de changement de mode détermine s'il est possible de commander le mode de commande du tube flexible basé sur les informations d'image en se fondant sur les informations d'image de la zone d'extrémité de l'instrument chirurgical.
